Europäisches Patentamt

European Patent Office

Office européen des brevets

(19)

(11) **EP 0 912 480 B1**

(12) **EUROPÄISCHE PATENTSCHRIFT**

(45) Veröffentlichungstag und Bekanntmachung des Hinweises auf die Patenterteilung:
**19.09.2001 Patentblatt 2001/38**

(21) Anmeldenummer: **97927059.2**

(22) Anmeldetag: **28.05.1997**

(51) Int Cl.⁷: **C07C 45/33**, C07C 47/22, C07C 51/25, C07C 57/04

(86) Internationale Anmeldenummer:
**PCT/EP97/02762**

(87) Internationale Veröffentlichungsnummer:
**WO 97/46506 (11.12.1997 Gazette 1997/53)**

(54) **GROSSTECHNISCHES VERFAHREN DER HETEROGEN KATALYSIERTEN GASPHASENOXIDATION VON PROPAN ZU ACROLEIN**

INDUSTRIAL PROCESS FOR THE HETEROGENEOUSLY CATALYTIC GAS-PHASE OXIDATION OF PROPANE TO FORM ACROLEINE

PROCEDE INDUSTRIEL POUR OXYDER EN PHASE GAZEUSE DU PROPANE EN ACROLEINE PAR CATALYSE HETEROGENE

(84) Benannte Vertragsstaaten:
**BE DE DK ES FR GB IT NL**

(30) Priorität: **04.06.1996 DE 19622331**

(43) Veröffentlichungstag der Anmeldung:
**06.05.1999 Patentblatt 1999/18**

(73) Patentinhaber: **BASF AKTIENGESELLSCHAFT**
**67056 Ludwigshafen (DE)**

(72) Erfinder:
• **TENTEN, Andreas**
**D-67487 Maikammer (DE)**
• **PROLL, Theo**
**D-67098 Bad Dürkheim (DE)**

• **SCHILDBERG, Hans-Peter**
**D-67433 Neustadt (DE)**

(56) Entgegenhaltungen:
**DE-A- 2 058 054**

• **DATABASE WPI Section Ch, Week 9018 Derwent Publications Ltd., London, GB; Class A41, AN 90-135680 XP002040183 & JP 02 083 348 A (MITSUBISHI PETROCHEMICAL CO LTD) , 23.März 1990 in der Anmeldung erwähnt**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein neues Verfahren der großtechnischen heterogen katalysierten Gasphasenoxidation von Propan zu Acrolein an Multimetalloxidmassen in einem Oxidationsreaktor, dessen Beschickungsgasgemisch neben Propan und molekularem Sauerstoff als Oxidationsmittel höchstens noch ein sich unter den Bedingungen der heterogen katalysierten Gasphasenoxidation im wesentlichen inert verhaltendes Verdünnungsgas umfaßt.

Acrolein ist ein bedeutendes Zwischenprodukt, beispielsweise für die Herstellung von Glutardialdehyd, Methionin, Folsäure und Acrylsäure.

Es ist seit langem bekannt, Acrolein großtechnisch durch heterogen katalysierte Gasphasenoxidation von Propylen mit molekularem Sauerstoff an im festen Aggregatzustand befindlichen Katalysatoren zu erzeugen (vgl. z. B. DE-A 19 62 431, DE-A 29 43 707, DE-PS 1 205 502, EP-A 257 565, EP-A 253 409, US-A 29 41 007 etc.).

Nachteilig an dieser Verfahrensweise ist, daß mit Propylen von einer relativ teuren Ausgangsverbindung ausgegangen wird.

Aus der EP-A 117 146 ist bekannt, Acrolein ausgehend von Propan dadurch zu erzeugen, daß man Propan in Abwesenheit von Sauerstoff an geeigneten Katalysatoren zunächst partiell dehydriert und anschließend das im resultierenden Produktgemisch enthaltene Propylen ohne Vorabtrennung gemäß vorstehend genannter heterogen katalysierter Gasphasenoxidation zu Acrolein umsetzt. Nachteilig an dieser Verfahrensweise ist das Erfordernis einer separaten Dehydrierstufe.

Inzwischen ist allgemein bekannt, Acrolein unmittelbar durch heterogen katalysierte Gasphasenoxidation von Propan mit molekularem Sauerstoff zu erzeugen (vgl. z.B. US-A 4 472 314; Wm. Curtis Conner Jr. und Stuart Soled, "Propane Oxidation over Mixed Metal Oxides", S. 1224-38 in Stud. Surf. Sci. Catal., 7 (1981); US-A 4 302 610; J. Barrault und L. Magaud, "Selective oxidation of propane in the presence of bismuth-based catalysts", S. 305-14 in Stud. Surf. Sci. Catal. 81 (1994);

Ikuya Matsuura und Naomasa Kimura, "Oxidation and ammoxidation of propane over tetragonal type $M^5+OPO_4$ catalysts", S. 271-79 in Stud. Surf. Sci. Catal. 82 (1994); Wu Tong-Hao et.al. in Journal of Natural Gas Chemistry, 1 (1994) S. 53-60; JP-A 6-199 731; Kim, Y.C. et al., Applied Catalysis, 70 (1991), S. 175-87; Kim, Y.C. et al., Chemistry Letters, 4 (1989) S. 531-34; JP-A-2-83348; Takita Y. et al., Chemistry Letters, 10 (1989) S. 1733-36; Kim, Y.C. et al., J. Chem. Soc., Chem. Commun. (1989) S. 652-53; Kim, Y.C. et al., Catalytic Science and Technology, Vol. 1, Kodanska Ltd. (1991) S. 439-40; Takita Y. et al., Catalysis Today, 13 (1992) S. 673-78; Y. Moro-oka, Stud. Surf. Sci. Catal. 75c (1993) S. 1983-86; US-A-4 260 822, GB-1340891 und US-A 3 293 290). Bei den diesbezüglich zu verwendenden Katalysatoren handelt es sich um im festen Aggregatzustand befindliche Oxidmassen. Die katalytisch aktive Oxidmasse kann neben Sauerstoff lediglich ein anderes Element oder mehr als ein anderes Element (Multimetalloxidmassen) enthalten. Besonders häufig kommen als katalytisch aktive Oxidmassen solche zur Anwendung, die mehr als ein metallisches, insbesondere übergangsmetallisches, Element umfassen. Üblicherweise sind diese Multielementoxidmassen keine einfachen physikalischen Gemische von Oxiden der elementaren Konstituanten, sondern heterogene Gemische von komplexen Polyverbindungen dieser Elemente.

Als besonders geeignete Katalysatoren für die gasphasenkatalytische Oxidation von Propan zu Acrolein haben sich Multimetalloxidmassen der allgemeinen Formel I

$$Mo_aBi_bP_cX^1_dX^2_eX^3_fX^4_gO_h \qquad\qquad (I),$$

mit

$X^1$ =    V, Nb, Ta, Cr, W, Ga, Ce und/oder La,
$X^2$ =    Li, Na, K, Rb, Cs, Cu, Ag, Au, Pd und/oder Pt,
$X^3$ =    Sn, Pb, Sb, Bi, Te, Fe, Co und/oder Ni,
$X^4$ =    Si, Al, Ti und/oder Zr,

```
a  =  0 bis 2,    │  mit der Maßgabe, daß die
d  =  0 bis 2,    │  Summe aus a und d wenigstens 0,20
                  │  beträgt;
b  =  0 bis 1,5,  │  mit der Maßgabe, daß die Summe aus
c  =  0 bis 10,   │  b und c wenigstens 0,1 beträgt;
```

e =    0 bis 0,5,

f =    0 bis 0,5,

g =    0 bis 20 und

h =    eine von Null verschiedene Zahl, die durch die Wertigkeit und Häufigkeit der von Sauerstoff verschiedenen Elemente in I bestimmt wird,

erwiesen.

Infolge der im Vergleich zu Propylen erhöhten Reaktionsträgheit von Propan erfolgt die heterogen katalysierte Gasphasenoxidation von Propan zu Acrolein bei vergleichsweise erhöhter Temperatur. Typischerweise beträgt die Reaktionstemperatur 350 bis 650°C. Da die Gasphasen-Partialoxidation des Propans exotherm verläuft, ist es zweckmäßig, ihre großtechnische Durchführung z.B. im Katalysator-Wirbelbett oder in Vielkontaktrohr-Festbettreaktoren durchzuführen, durch deren die Kontaktrohre umgebenden Raum ein Wärmeaustauschmittel (z.B. Salzbad oder Metallschmelze) geleitet wird. Der Arbeitsdruck (Absolutdruck) bei der großtechnischen heterogen katalysierten Gasphasen-Partialoxidation von Propan kann dabei sowohl unter 1 atm, bei 1 atm oder über 1 atm liegen. In der Regel beträgt er 1 bis 2 atm, er kann jedoch auch bis zu 10 atm betragen. Die Zielumsetzung erfolgt während der Verweilzeit des Reaktionsgemisches in der Katalysatorbeschickung, durch die es geleitet wird.

Im Vordergrund der großtechnischen heterogen katalysierten Gasphasen-Partialoxidation von Propan mit molekularem Sauerstoff steht einerseits das Interesse an einer hohen Raum-Zeit-Ausbeute der gewünschten Zielverbindung Acrolein. Aufgrund der zum Vergleich zu Propylen erhöhten Reaktionsträgheit von Propan wird daher im Stand der Technik für die gasphasenkatalytische Partialoxidation des Propans, im Unterschied zur gasphasenkatalytischen Partialoxidation des Propylens, mehrheitlich empfohlen, ein Reaktionsgasausgangsgemisch einzusetzen, das das Propan bezüglich seines Reaktionspartners, dem molekularen Sauerstoff, im Überschuß aufweist. Andererseits weist die in Rede stehende Partialoxidation des Propans einen ausgeprägt exothermen Charakter auf, weshalb bezüglich der großtechnischen gasphasenkatalytisch oxidativen Umsetzung von Propan zu Acrolein im Stand der Technik empfohlen wird, entweder den Volumenanteil des Propans im Reaktionsgasausgangsgemisch nach oben zu beschränken, oder die Reaktionspartner mit einem sich unter den Bedingungen der gasphasenkatalytischen Propanpartialoxidation im wesentlichen inert verhaltenden Gas zu verdünnen. Als ein sich unter den Bedingungen der heterogen katalysierten Gasphasen-Partialoxidation im wesentlichen inert verhaltendes Verdünnungsgas werden dabei normalerweise solche Verdünnungsgase verstanden, deren Bestandteile unter den Bedingungen der heterogen katalysierten Gasphasen-Partialoxidation - jeder Bestandteil für sich betrachtet - zu wenigstens 97 mol-% unverändert bleiben. Beispiele für solche im Stand der Technik für die großtechnische gasphasenkatalytische Partialoxidation des Propans empfohlene inerte Verdünnungsgase sind $N_2$, $CO_2$, CO, Edelgase und Wasserdampf. Eine besonders wichtige vorteilhafte Wirkung der Mitverwendung eines inerten Verdünnungsgases wird im Stand der Technik darin gesehen, daß, bei einem vorgegebenen Sauerstoff-zu-Propan-Verhältnis, der Zusatz eines inerten Verdünnungsgases die Explosionsneigung des Gasgemisches mindert. D.h., der Zusatz eines inerten Verdünnungsgases erhöht den für eine im Gasgemisch sich selbständig ausbreitende Verbrennungsreaktion erforderlichen Energieeintrag, was für die Sicherheit einer großtechnischen Fahrweise von herausragender Bedeutung ist. Zusätzlich schreibt der Stand der Technik den inerten Verdünnungsgasen eine vorteilhafte Wirkung im Hinblick auf die Selektivität der Produktbildung zu.

Im Stand der Technik ist, mit Ausnahme der DE-OS 22 14 214 (s.u.), keine großtechnische katalytische Gasphasenpartialoxidation von Propan mit molekularem Sauerstoff zu Acrolein beschrieben, bei der das Beschickungsgasgemisch (Reaktionsgasausgangsgemisch) mehr als 70 Vol.-% Propan aufweist.

Vorstehende Aussage trifft insbesondere auch auf die EP-A 609 122, EP-A 10902 sowie auf die CN-A 1 105 352 zu und in der JP-A 2/83 384 wird sogar dezidiert von der Anwendung eines oberhalb 60 Vol-% liegenden Propananteils im Reaktionsgasausgangsgemisch abgeraten (die Anwendung eines mehr als 70 Vol-% Propan aufweisenden Beschickungsgasgemisches wird in der Literatur im Zusammenhang mit der katalytischen Gasphasenoxidation von Propan mit molekularem Sauerstoff zu Acrolein lediglich von der Autorengruppe um A.N. Schatalowa in Neftechimija 8 (1968), Nr. 3, S. 364-369 bzw. dem dazu äquivalenten russischen Urheberschein 176878 (Bekanntmachung 1966) berichtet; diese Veröffentlichungen betreffen jedoch keine großtechnischen Verfahren zur Herstellung von Acrolein, sondern beschränken sich auf Laborversuche zur Testung von Katalysatoren; im Rahmen derartiger Kleinversuche ist die Frage der Explosionsneigung des Reaktionsgemisches aber von untergeordneter Bedeutung; dies weist auch die hohe angewandte Reaktionstemperatur von 600°C aus).

Die DE-OS 22 14 214 beschreibt ein Oxidationsverfahren von gesättigten Paraffinkohlenwasserstoffen, bei denen das Volumenverhältnis von Sauerstoff, beispielsweise in Form von Luft eingesetzt, zum gesättigten Paraffinkohlenwasserstoff vorzugsweise zwischen 1:3 und 1:9 liegt.

Nachteilig an der Lehre des Standes der Technik ist jedoch, daß bei vorgegebenem Katalysator und Reaktionstemperatur weder die Selektivität der Acroleinbildung, noch der bei einmaligem Reaktordurchgang erzielte Umsatz an Propan, noch die Explosionsneigung des Reaktionsgasausgangsgemisches voll zu befriedigen vermag.

Die Aufgabe der vorliegenden Erfindung bestand daher darin, ein verbessertes großtechnisches Verfahren der heterogen katalysierten Gasphasenoxidation von Propan zu Acrolein an Multimetalloxidmassen in einem Oxidationsreaktor, dessen Beschickungsgasgemisch neben Propan und molekularem Sauerstoff als Oxidationsmittel höchstens noch ein sich unter den Bedingungen der heterogen katalysierten Gasphasenoxidation im wesentlichen inert verhaltendes Verdünnungsgas umfaßt, zur Verfügung zu stellen.

Demgemäß wurde ein Verfahren der großtechnischen heterogen katalysierten Gasphasenoxidation von Propan zu Acrolein in einem Oxidationsreaktor, dessen Beschickungsgasgemisch neben Propan und molekularem Sauerstoff als Oxidationsmittel höchstens noch ein sich unter den Bedingungen der heterogen katalysierten Gasphasenoxidation im wesentlichen inert verhaltendes Verdünnungsgas umfaßt, umfaßt und dessen Beschickungsgasgemisch zu mehr als

70 Vol.-% aus Propan und zu wenigstens 5 Vol.-% aus molekularem Sauerstoff besteht, gefunden, dadurch gekennzeichnet, daß als Katalysator eine Multimetalloxidmasse der allgemeinen Formel I

$$Mo_a Bi_b P_c X^1_d X^2_e X^3_f X^4_g O_h \qquad (I),$$

mit

$X^1 =$ V, Nb, Ta, Cr, W, Ga, Ce und/oder La,
$X^2 =$ Li, Na, K, Rb, Cs, Cu, Ag, Au, Pd und/oder Pt,
$X^3 =$ Sn, Pb, Sb, Bi, Te, Fe, Co und/oder Ni,
$C^4 =$ Si, Al, Ti und/oder Zr,

```
     a   = 0 bis 2     |   mit der Maßgabe, daß die Summe aus a
     d   = 0 bis 2,    |   und d wenigstens 0,20 beträgt;


     b   = 0 bis 1,5   |   mit der Maßgabe, daß die Summe aus b und c
     c   = 0 bis 10,   |   wenigstens 0,1 beträgt;
```

e = 0 bis 0,5,
f = 0 bis 0,5,
g = 0 bis 20 und
h = eine von Null verschiedene Zahl, die durch die Wertigkeit und Häufigkeit der von Sauerstoff verschiedenen Elemente in I bestimmt wird,

geformt zu einem Vollkatalysatorhohlzylinder mit einem Außendurchmesser und einer Länge von 2 bis 10 mm und einer Wandstärke von 1 bis 3 mm mitverwendet wird.

Als weitere Reaktionsausgangsgemischbestandteile kommen die bereits beispielhaft genannten inerten Verdünnungsgase $N_2$, $CO_2$, CO, Edelgase wie z.B. He und/oder Wasserdampf in Betracht. Bevorzugt ist deren Anteil im Beschickungsgasgemisch jedoch gering.

Unter einem großtechnischen Verfahren der heterogen katalysierten Gasphasenoxidation von Propan zu Acrolein mit molekularem Sauerstoff in einem Oxidationsreaktor soll in dieser Schrift ein solches Verfahren verstanden werden, bei dem die Belastung des Oxidationsreaktors mit Beschickungsgasgemisch (Reaktionsgasausgangsgemisch) wenigstens 500 $Nm^3$/h beträgt ($Nm^3$ = Normkubikmeter, d.h. auf 1 atm und 25°C bezogen). In der Regel wird vorstehende Belastung wenigstens 700 $Nm^3$/h und häufig wenigstens 1000 oder 5000 $Nm^3$/h betragen. Eine Belastung von 100000 $Nm^3$/h wird im Normalfall nicht überschritten werden. D.h., typische großtechnische Belastungen liegen bei 10000 bis 60000 $Nm^3$/h.

Als zu verwendende Katalysatoren kommen für das erfindungsgemäße Verfahren, bei Anwendung vorgenannter Reaktionsgasausgangsgemische, die Multimetalloxidmassen der allgemeinen Formel I in Betracht, wie sie in den den Stand der Technik wiedergebenden eingangs zitierten Schriften beschrieben sind.

Bevorzugt werden als erfindungsgemäß zu verwendende Multimetalloxidmassen diejenigen der allgemeinen Formel II

$$Mo_a, Bi_b, X^5_c, X^6_d, X^7_e, O_f, \qquad (II),$$

mit

X⁵ = $X^5 =$   V, Nb, Ce, Ta, Fe, Ga und/oder P, vorzugsweise V, Nb und/ oder Ce, und besonders bevorzugt V und/oder Nb,

$X^6 =$   Ag, Li, Na, K, Rb, Cs, Tl, Pd, Pt, Au, Cu, Pb und/oder Te, vorzugsweise Ag, Li und/oder Na und besonders bevorzugt Ag,

$X^7 =$   Si, Al, Ti und/oder Zr,

$a' =$   0,2 bis 2,

$b' =$   0,3 bis 1,5,

$c' =$   0 bis 2, vorzugsweise 0,2 bis 1,

$d' =$   0 bis 0,5, vorzugsweise 0,001 bis 0,1,

$e' =$   0 bis 20 und

$f' =$   eine von Null verschiedene Zahl, die durch die Wertigkeit und Häufigkeit der von Sauerstoff verschiedenen Elemente in II bestimmt wird,

sowie diejenigen der allgemeinen Formel III

$$X^8{}_1 P_{a''} X^9{}_{b''} X^{10}{}_{c''} O_{d''} \qquad (III),$$

mit

$X^8 =$   V, Nb, Ta, Cr, Mo und/oder W, vorzugsweise V, Nb und/oder Ta,

$X^9 =$   Sn, Sb, Bi, Te, Fe, Co, Ni, Cu, La und/oder Ce, vorzugsweise Sn, Sb, Bi und/oder Te,

$X^{10} =$   Li, Na, K, Rb und/oder Cs,

$a'' =$   1 bis 10,

$b'' =$   0 bis 0,5,

$c'' =$   0 bis 0,5 und

$d'' =$   eine von Null verschiedene Zahl, die durch die Wertigkeit und Häufigkeit der von Sauerstoff verschiedenen Elemente in III bestimmt wird,

eingesetzt.

Die Herstellung dieser Multimetalloxidmassen I bis III ist dem Fachmann bekannt und im Stand der Technik beschrieben. In typischer Weise wird aus geeigneten Quellen der elementaren Konstituenten der Multimetalloxidmasse ein inniges Trockengemisch erzeugt, das in der Regel bei einer Temperatur von 450 bis 650°C einige Stunden calciniert wird. In einfachster Weise wird die Calcination an Luft durchgeführt. Abschließend wird die im Rahmen der Calcination resultierende Aktivmasse entweder als solche eingesetzt, oder in an sich bekannter Weise zur gewünschten Katalysatorgeometrie geformt. Natürlich kann die Formgebung auch vorab der Calcination erfolgen.

Die erfindungsgemäßen Multimetalloxidmassen werden als Vollkatalysatoren betrieben. Diesbezüglich wird das innige Trockengemisch vorzugsweise unmittelbar zur gewünschten Katalysatorgeometrie verdichtet (z.B. Tablettieren, Extrudieren oder Strangpressen), wobei gegebenenfalls an sich übliche Hilfsmittel, z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können, und calciniert. Generell kann auch hier vor der Formgebung calciniert werden. Die geeignete Vollkatalysatorgeometrie sind Hohlzylinder mit einem Außendurchmesser und einer Länge von 2 bis 10 mm und einer Wandstärke von 1 bis 3 mm. Wesentlich für die Quellen der elementaren Konstituenten der Multimetalloxidmassen I bis III ist dabei, wie allgemein bekannt, nur, daß es sich entweder bereits um Oxide handelt oder um solche Verbindungen, die durch Erhitzen, wenigstens in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht. Geeignete Ausgangsverbindungen des Mo, V, W und Nb sind auch deren Oxoverbindungen (Molybdate, Vanadate, Wolframate und Niobate) bzw. die von diesen abgeleiteten Säuren.

Das innige Mischen der Ausgangsverbindungen im Rahmen der Herstellung von erfindungsgemäß zu verwendenden Multimetalloxidmassen kann in trockener oder in nasser Form erfolgen. Erfolgt es in trockener Form, werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden die Ausgangsverbindungen dabei in Form einer wäßrigen Lösung und/oder Suspension miteinander vermischt. Beispielsweise kann der Trocknungsprozeß unmittelbar im Anschluß an die Fertigstellung der wäßrigen Mischung und durch Sprühtrocknung (die Austrittstemperaturen betragen zweckmäßigerweise 100 bis 150°C) erfolgen, die ein besonders inniges Trockengemisch bedingt.

Erfindungsgemäß wesentlich ist, daß das erfindungsgemäße Verfahren auch mit Multimetalloxidmassen I, II und/oder III ausnähmlich solcher Multimetalloxidmassen, die dadurch erhältlich sind, daß man dehydrierende Oxide wie Magnesium-, Zink-, Aluminium-, Chrom-, Kupfer-, Kalium- und/oder Eisenoxid mit einem oxidischen Wismut-Molybdat innig vermischt (gegebenenfalls unter Zusatz einer geringen Wassermenge) und (gegebenenfalls nach Trocknung) bei erhöhter Temperatur (z.B. ca. 600°C) härtet, anwendbar ist.

D.h., das erfindungsgemäße Verfahren gelingt auch mit Multimetalloxidmassen I, II und/oder III ausnähmlich solcher Multimetalloxidmassen, die ein dehydrierendes Metalloxid wie Magnesium-, Zink-, Aluminium-, Chrom-, Kupfer-, Kalium- und/oder Eisenoxid enthalten, wie sie in den als Stand der Technik zitierten Veröffentlichung von A.N. Schatalowa verwendet wurden.

Die Reaktionstemperatur in Anwendung des erfindungsgemäßen großtechnischen Verfahrens beträgt zweckmäßigerweise 350 bis 650°C. Einen besonders hohen Sicherheitsstandard gewährleistet das erfindungsgemäße Verfahren bei Temperaturen $\leq 550°C$, vorzugsweise $\leq 500°C$. Da sich Temperaturen $< 400°C$ bereits nachteilig auf den Umsatz auswirken, liegt der erfindungsgemäß günstigste Temperaturbereich somit bei 400 bis 550°C, vorzugsweise bei 400 bis 500°C und besonders bevorzugt bei 425 bis 475°C.

Diese Aussagen treffen insbesondere dann zu, wenn als Katalysatoren Multimetalloxidmassen der allgemeinen Formeln I, II und/oder III angewendet werden.

Selbstverständlich kann das erfindungsgemäß zu verwendende Reaktionsgasausgangsgemisch der Katalysatorbeschickung bereits vorerwärmt zugeführt werden. Dabei ist es nicht von Nachteil, wenn das Reaktionsgasausgangsgemisch der Katalysatorbeschickung bereits auf die gewünschte Reaktionstemperatur vorerwärmt zugeführt wird. Dazu kann dem eigentlichen Oxidationsreaktor eine Aufheizvorrichtung vorgeschaltet sein.

Im Fall einer Anwendung von Vielkontaktrohr-Festbettreaktoren, wie sie z.B. in den Schriften DE-A 28 30 765, DE-A 22 01 528 oder US-A 3 147 084 beschrieben sind, kann eine solche vorgeschaltete Aufheizvorrichtung z. B. in besonders einfacher Weise aus einem an Katalysator freien, auf Reaktionstemperatur beheizten, Kontaktrohrabschnitt bestehen. Dieser Abschnitt kann aber auch an Kontaktrohren frei sein und so einem bis zu Reaktorquerschnitt aufweisenden (Einzel) rohr entsprechen. Eine im Rahmen einer solchen Vorerwärmung einhergehende partielle thermische und/oder oxidative Dehydrierung des Propans zu Propylen beeinträchtigt den Erfolg des erfindungsgemäßen Verfahrens nicht. Dies trifft auch dann zu, wenn sich im Rahmen einer solchen Vorerwärmung die entsprechenden zugehörigen Zerfallgleichgewichte einstellen.

Für das erfindungsgemäße großtechnische Verfahren geeignete Vielkontaktrohr-Festbettreaktoren enthalten wenigstens 1000 Einzelrohre. In der Regel werden sie nicht mehr als 50000 Einzelrohre enthalten. Typisch ist eine Anzahl von wenigstens 5000 bzw. wenigstens 10000 Einzelrohren. Häufig beträgt die Anzahl der im Reaktionsbehälter untergebrachten Kontaktrohre 15000 bis 30000. Solche Vielkontaktrohr-Festbettreaktoren entsprechen in ihrem Typ den Mantel-Rohr-Wärmeaustauschern. D.h., ihre einfachste Bauart besteht aus einem, in der Regel zylinderförmigen, Behälter, in welchem eine Vielzahl von Rohren (ein Rohrbündel) entsprechend den Kühlrohren eines Mantel-Rohr-Wärmeaustauschers in üblicherweise vertikaler Anordnung untergebracht ist. Ferner werden durch den die Kontaktrohre umgebenden Raum Wärmeaustauschmittel geleitet, um die Prozeßwärme zu bewältigen. Üblicherweise sind die Kontaktrohre aus ferritischem Stahl gefertigt und weisen in typischer Weise eine Wanddicke von 1 bis 3 mm auf. Ihr Innendurchmesser beträgt in der Regel 20 bis 30 mm. Die Rohrlänge erstreckt sich im Normalfall auf wenige Meter (typisch ist eine Kontaktrohrlänge im Bereich von 2 bis 4 m). Innerhalb des Reaktors sind die Kontaktrohre im Normalfall homogen verteilt angeordnet.

Wie bereits eingangs erwähnt, vermag sich der Arbeitsdruck des erfindungsgemäßen Verfahrens über einen weiten Bereich (typisch 0,5 atm bis 10 atm) zu erstrecken. Mit Vorteil beträgt der Arbeitsdruck 1 bis 3, vorzugsweise 1 bis 2 atm. Die Gesamtraumbelastung wird bei Anwendung von Vielkontaktrohr-Festbettreaktoren für das erfindungsgemäße Verfahren in der Regel auf Werte von 200 bis 3500 Nl/1/h eingestellt.

Aus dem erfindungsgemäß erhältlichen Produktgasgemisch kann das gebildete Acrolein in an sich bekannter Weise abgetrennt werden. Nicht umgesetztes Propan und gegebenenfalls gebildetes Propylen kann in die Oxidationsstufe rückgeführt (kontinuierliche Verfahrensweise) und/oder anderweitig (z.B. Verbrennung in Wärmekraftwerken zur Energieerzeugung, Herstellung von Synthesegas oder Acetylen etc.) weiterverwendet werden. Bei einer Rückführung in die Oxidationsstufe werden in der Regel in der Oxidationsstufe als Nebenprodukte gebildete inerte Verdünnungsgase wie $CO_2$, $CO$, $H_2O$ etc. wenigstens teilweise abgetrennt, um die erfindungsgemäß erforderliche Zusammensetzung des Reaktionsgasausgangsgemisches nicht zu beeinträchtigen.

Eine andere Weiterverwendungskaskade des erfindungsgemäß resultierenden Produktgasgemisches bietet die an sich bekannte katalytische Gasphasenoxidation des darin enthaltenen Acroleins zu Acrylsäure. Dazu kann das erfindungsgemäß anfallende Produktgasgemisch in einfachster Weise als solches in eine entsprechende nachfolgende Gasphasenoxidationsstufe geführt werden. Erfindungsgemäß von Vorteil ist dabei, daß das im Produktgasgemisch enthaltene nicht umgesetzte Propan in einer solchen nachfolgenden Gasphasenoxidationsstufe ebenfalls als die Explosionsneigung minderndes Verdünnungsgas wirkt und auch in diesem Bereich die Verfahrenssicherheit erhöht. Die einfachste Realisierungsform dieser zweiten Oxidationsstufe bildet eine Verlängerung der Katalysatorschüttung der ersten Oxidationsstufe. Eine andere einfache Realisierungsform der beiden Oxidationsstufen bildet ein Rohrbündelreaktor, innerhalb dessen sich die Katalysatorbeschickung längs der einzelnen Kontaktrohre mit Beendigung des ersten Reaktionsschrittes entsprechend ändert. Die beiden Oxidationsstufen können jedoch auch in Form eines aus zwei hintereinandergeschalteten Oxidationsreaktoren (für die Acrylsäurestufe können entsprechende Rohrbündelreaktoren verwendet werden wie für die Acroleinstufe beschrieben; sie sind im Stand der Technik bekannt; vgl. z.B. DE-A 4 431 949 und darin zitierte Literatur;) bestehenden Oxidationsreaktors realisiert sein. In diesem Fall können auch die übrigen Reaktionsbedingungen, z.B. die Reaktionstemperatur, in einfacher Weise optimierend angepaßt werden. Zweckmäßigerweise führt man in diesem Fall den für die zweite Oxidationsstufe benötigten molekularen Sauerstoff erst dem zweiten Oxidationsreaktor zu. Die Menge an zusätzlich zugeführtem Sauerstoff wird vorzugsweise so gewählt, daß das Beschickungsgasgemisch der Acrylsäurestufe eine wenigstens stöchiometrische bis etwa dreifach stöchiometri-sche Menge an $O_2$ umfaßt. Die Sicherheit für die zweite Oxidationsstufe (Acrylsäurestufe) kann man zusätzlich dadurch erhöhen, daß man aus dem die erste Oxidationsstufe verlassenden Produktgasgemisch in der ersten Oxidationsstufe als Nebenprodukte gebildeten Wasserdampf und $CO_2$ abtrennt, bevor man es in die Acrylsäurestufe überführt. Selbstverständlich kann diesbezüglich das enthaltende Acrolein aber auch zuvor vollständig oder wenigstens von einem Teil (z.B. von CO, $CO_2$, $H_2O$, $N_2$) der ansonsten im Produktgemisch enthaltenen Gaskomponenten abgetrennt werden. Selbstredend kann das Produktgasgemisch vor einer solchen Weiterverwendung zur Acrylsäureherstellung aber auch durch externen Zusatz gasförmiger Komponenten (z.B. Wasserdampf) in seiner Zusammensetzung wunschgemäß verändert werden. Im Produktgasgemisch einer solchen nachgeschalteten Acrylsäurestufe noch enthaltenes Propan und/oder Acrolein kann ebenfalls abgetrennt und in die Acrolein- und/oder Acrylsäurestufe rückgeführt und/oder einer anderweitigen Verwendung (z.B. Verbrennung in Wärmekraftwerken zur Energieerzeugung, Herstellung von Synthesegas oder Acethylen etc.) zugeführt werden.

Die Reaktionstemperatur in einer solchen nachgeschalteten Acrylsäurestufe beträgt vorteilhaft 200 bis 350°C, vorzugsweise 230 bis 330°C (zwischen beide Oxidationsstufen wird zweckmäßig ein entsprechender Wärmetauscher geschaltet). Die in einer solchen Acrylsäurestufe angewandten Multimetalloxidkatalysatoren sind die in an sich bekannter Weise anzuwendenen Mo und V als Hauptbestandteile enthaltenden Multimetalloxide. Solchermaßen geeignete Multimetalloxidkatlysatoren können beispielsweise den US-A 3 775 474, US-A 3 954 855, US-A 3 893 951 und US-A 4 339 355 entnommen werden. Ferner eignen sich in besonderer Weise die Multimetalloxidmassen der EP-A 427 508, DE-A 2 909 671, DE-C 3 151 805, DE-AS 2 626 887 und der DE-A 4 302 991.

Eine Vielzahl der für die Acrylsäurestufe geeigneten Multimetalloxidkatalysatoren läßt sich unter der allge-meinen Formel IV

$$Mo_{12}V_{a'''}\,W_{b'''}Cu_{c'''}Ni_{d'''}X^{11}_{e'''}X^{12}_{f'''}X^{13}_{g'''}\,X^{14}_{h'''}\,X^{15}_{i'''}O_{n'''} \tag{IV},$$

in der die Variablen nachfolgende Bedeutung aufweisen:

$X^{11}$ ein oder mehrere Alkalimetalle,
$X^{12}$ ein oder mehrere Erdalkalimetalle,
$X^{13}$ Chrom, Mangan, Cer und/oder Niob,
$X^{14}$ Antimon und/oder Wismut,
$C^{15}$ Silicium, Aluminium, Titan und/oder Zirkonium,
a''' 1 bis 6,
b''' 0,2 bis 4,
c''' 0,5 bis 6,
d''' 0,2 bis 6,
e''' 0 bis 2,
f''' 0 bis 3,
g''' 0 bis 5,
h''' 0 bis 40,
i''' 0 bis 40 und
n''' eine Zahl, die durch die Wertigkeit und Häufigkeit der von Sauerstoff verschiedenen Elemente bestimmt wird,

subsummieren.

Sie sind in an sich bekannter Weise erhältlich (siehe z.B. DE-A 4 302 991) und werden üblicherweise in Substanz zu Kugeln, Ringen oder Zylindern geformt oder auch in Gestalt von Schalenkatalysatoren, d.h., mit der Aktivmasse beschichteten vorgeformten inerten Trägerkörpern, eingesetzt. Selbstverständlich können sie aber auch in Pulverform als Katalysatoren angewendet werden.

Als Quelle für den im Rahmen des erfindungsgemäßen Verfahrens als Oxidationsmittel benötigten molekularen Sauerstoff kann z.B. Luft verwendet werden. Da molekularer Sauerstoff in Luft aber nur mit dem inerten Gas $N_2$ vergesellschaft vorkommt und erfindungsgemäß vorteilhaft ohne Beisein von Inertgasen im Reaktionsgasgemisch gearbeitet wird, wird man den für das erfindungsgemäße Verfahren benötigten Sauerstoff normalerweise einer reinen Sauerstoffquelle entnehmen.

Umsatz und Selektivität sind in dieser Schrift, falls nichts anderes erwähnt wird, wie folgt definiert:

$$\text{Umsatz U an Propan bei einmaligem Durchgang} \quad (\text{mol-\%}) = \frac{\text{Molzahl umgesetztes Propan}}{\text{Molzahl eingesetztes Propan}} \times 100;$$

$$\text{Selektivität S der Acroleinbildung bei einmaligem Durchgang} \quad (\text{mol-\%}) = \frac{\text{Molzahl Propan umgesetzt zu Acrolein}}{\text{Molzahl Propan insgesamt umgesetzt}} \times 100;$$

Beispiele

A) Herstellung von erfindungsgemäß als Katalysatoren zu verwendenden Multimetalloxidmassen MI bis MIII

MI: 413,3 g Bismutnitratpentahydrat ($Bi(NO_3)_3 \cdot 5H_2O$, der Fa. Merck, Darmstadt) wurden bei 25°C in 400 ml Wasser gelöst und mit 40 ml konzentrierter Salpetersäure (65 gew.-%ige wäßrige Lösung) zu einer wäßrigen Lösung A angesäuert.

Weiterhin wurden in 800 ml Wasser bei 95°C zunächst 79,31 g Ammoniumheptamolybdattetrahydrat (81,7 Gew.-% $MoO_3$) und danach 65,29 g Ammoniummetavanadat (75,2 Gew.-% $V_2O_5$) gelöst. Die resultierende Lösung wurde auf 80°C abgekühlt und anschließend durch Zusatz von 47 ml einer 25 gew.-%igen wäßrigen $NH_3$-Lösung eine einen pH-Wert von 10 aufweisende wäßrige Lösung B erzeugt. Außerdem wurden 1,7 g Silbernitrat in 5 ml Wasser zu einer wäßrigen Lösung C gelöst.

Dann wurden die wäßrigen Lösungen A und C in der genannten Abfolge (unter Aufrechterhaltung der 80°C) in die 80°C aufweisende wäßrige Lösung B unter kräftigem Rühren eingebracht, die resultierende wäßrige Suspension nach 1 h unter Aufrechterhaltung der 80°C gerührt und anschließend auf einem 80°C aufweisenden Wasserbad unter Rühren bei Normaldruck (1 atm) bis zur breiförmigen Konsistenz langsam eingedampft. Danach wurde die dabei resultierende Masse noch 16 h bei 120°C und 1 atm getrocknet sowie abschließend zu einen zahlenmittleren Größtdurchmesser von 5 mm aufweisenden Katalysatorvorläuferteilchen gebrochen. Letztere wurden abschließend 6 h bei 520°C an Luft calciniert und zu einen zahlenmittleren Durchmesser von 150 µm aufweisenden Aktivmassenpartikeln gemahlen.

Die Stöchiometrie der Aktivmassenpartikel lautete:

$Bi_{0,85}V_{0,54}Mo_{0,45}Ag_{0,01}O_x$

MII: 181,9 g Vanadiumpentoxid ($V_2O_5$, > 99 gew.-%ig der Fa. Merck, Darmstadt) wurden unter Rühren in eine Mischung. aus 850 ml Wasser und 230,5 g 85 gew.-%iger wäßriger Phosphorsäure ($H_3PO_4$) eingetragen. Die resultierende wäßrige Suspension wurde bei Normaldruck während 5 h bei 100°C unter Rühren und Rückfluß gehalten und anschließend bei Normaldruck auf einem 80°C aufweisenden Wasserbad bis zur breiförmigen Konsistenz eingedampft. Die resultierende Masse wurde bei Normaldruck während 16 h bei einer Temperatur von 120°C getrocknet sowie abschließend zu einen zahlenmittleren Größtdurchmesser von 5 mm aufweisenden Katalysatorvorläuferteilchen gebrochen. Letztere wurden abschließend 3 h bei 500°C an Luft calciniert und zu einen zahlenmittleren Größtdurchmesser von 1 bis 2 mm aufweisenden Aktivmassenpartikeln versplittet.

Die Stöchiometrie der Aktivmassenpartikel lautete: $VOPO_4$.

MIII: In 1000 ml Wasser wurden bei 25°C 500 g $H_3PMo_{12}O_{40}$-Hydrat (50 Gew.-% Mo, der Fa. Merck, Darmstadt) gelöst. Zu dieser Lösung wurden unter Rühren innerhalb von 15 min 3270,6 g einer wäßrigen $Bi(NO_3)_3$-Lösung (11,1 Gew.-% Bi, $HNO_3$-sauer mit pH = 0,3) getropft. Die resultierende wäßrige Suspension wurde bei Normaldruck auf einem 70°C aufweisenden Wasserbad unter Rühren bis zur breiförmigen Konsistenz eingedampft. Die dabei anfallende Masse wurde danach noch 16 h bei 120°C und Normaldruck getrocknet sowie abschließend zu einen zahlenmittleren Größtdurchmesser von 5 bis 6 mm aufweisenden Katalysatorvorläuferpartikeln gebrochen. Diese wurden an Luft zunächst 4,5 h bei 460°C und danach noch 2 h bei 520°C calciniert und zu einen zahlenmittleren Durchmesser von 1 bis 2 mm aufweisenden Aktivmassenpartikeln versplittet.

Die Stöchiometrie der Aktivmassenpartikel lautete: $Bi_8PMo_{12}O_x$.

B) Durchführung katalytischer Gasphasenoxidation von Propan unter Verwendung der Multimetalloxidmassen MI bis MIII als Katalysatoren

V1: In ein einen Innendurchmesser von 17 mm aufweisendes Reaktionsrohr aus V2A Stahl (Wanddicke: 2 mm), das eine Länge von 25 cm und in seinem Zentrum längs des gesamten Rohres eine einen Außendurchmesser von 3 mm aufweisende Thermohülse aufwies, wurde auf ein das Reaktionsrohr abschließendes engmaschiges Tragenetz aus V2A Stahl eine Aktivmassenbeschickung aus 2 g der Aktivmassenpartikel MI und 10 g Steatitkugeln (2 mm Durchmesser, dienten zur Verdünnung der Aktivmasse) aufgebracht. Das Reaktionsrohr selbst war auf seiner gesamten Länge von einem Salzbad umgeben, das eine Temperatur von 450°C aufwies. Von dem dem Tragenetz entgegengesetzten Ende des Reaktionsrohres herkommend, wurde das Reaktionsrohr mit 6 N1/h an 25°C aufweisenden Reaktionsgasausgangsgemischen beschickt. Das das Kontaktrohr verlassende Produktgasgemisch wurde on-line gaschromatographisch analysiert.
In Abhängigkeit von der Zusammensetzung Z des Reaktionsgasausgangsgemisches wurden die nachfolgenden Ergebnisse erzielt:

| Z | | | | |
|---|---|---|---|---|
| $N_2$ (Vol.-%) | Propan (Vol.-%) | $O_2$ (Vol.-%) | $CO_2$ (Vol.-%) | S Acrolein, mol.-% |
| - | 33 | 67 | - | 5 |
| - | 50 | 50 | - | 6 |
| - | 75 | 25 | - | 8 |
| - | 80 | 20 | - | 9 |
| 10 | 45 | 45 | - | 8 |
| - | 45 | 45 | 10 | 8 |

Es überrascht, daß mit zunehmendem Propananteil des Beschickungsgasgemisches die Selektivität der Acroleinbildung zunimmt.

V2: Wie V1, die Aktivmassenbeschickung bestand jedoch aus einem Gemisch aus 4 g der Aktivmassenpartikel MII und 20 g der Steatitkugeln. Ferner betrug die Beschickung mit aus Propan und $O_2$ bestehendem Reaktionsgasausgangsgemisch 20 N1/h und die Salzbadtemperatur betrug 480°C.
Die erzielten Ergebnisse lauten:

| z | | |
|---|---|---|
| Propan (Vol.-%) | $O_2$ (Vol.-%) | S (Acrolein, mol.-%) |
| 67 | 33 | 6 |
| 80 | 20 | 9 |

V3: Wie V1, die Aktivmassenbeschickung bestand jedoch ausschließlich aus 4 g der Aktivmassenpartikel III.
Die erzielten Ergebnisse lauten:

| Z | | |
|---|---|---|
| Propan (Vol.-%) | $O_2$ (Vol.-%) | S (Acrolein, mol.-%) |
| 67 | 33 | 5 |
| 80 | 20 | 8 |

**Patentansprüche**

1. Verfahren der großtechnischen heterogen katalysierten Gasphasenoxidation von Propan zu Acrolein in einem Oxidationsreaktor, dessen Beschickungsgasgemisch neben Propan und molekularem Sauerstoff als Oxidationsmittel höchstens noch ein inertes Verdünnungsgas umfaßt und dessen Beschickungsgasgemisch zu mehr als 70 Vol.-% aus Propan und zu wenigstens 5 Vol.-% aus molekularem Sauerstoff besteht, **dadurch gekennzeichnet, daß** als Katalysator eine Multimetalloxidmasse der allgemeinen Formel I

$$Mo_a Bi_b P_c X^1_d X^2_e X^3_f X^4_g O_h \qquad (I),$$

mit

$X^1 =$ V, Nb, Ta, Cr, W, Ga, Ce und/oder La,
$X^2 =$ Li, Na, K, Rb, Cs, Cu, Ag, Au, Pd und/oder Pt,
$X^3 =$ Sn, Pb, Sb, Bi, Te, Fe, Co und/oder Ni,
$X^4 =$ Si, Al, Ti und/oder Zr,

$$a = 0 \text{ bis } 2, \left. \right\} \text{ mit der Maßgabe, daß die Summe}$$
$$d = 0 \text{ bis } 2, \left. \right. \text{ aus a und d wenigstens 0,20 beträgt;}$$

$$b = 0 \text{ bis } 1,5, \left. \right\} \text{ mit der Maßgabe, daß die Summe aus}$$
$$c = 0 \text{ bis } 10, \left. \right. \text{ b und c wenigstens 0,1 beträgt;}$$

$e =$ 0 bis 0,5,
$f =$ 0 bis 0,5,
$g =$ 0 bis 20 und
$h =$ eine von Null verschiedene Zahl, die durch die Wertigkeit und Häufigkeit der von Sauerstoff verschiedenen Elemente in I bestimmt wird,

geformt zu einem Vollkatalysatorhohlzylinder mit einem Außendurchmesser und einer Länge von 2 bis 10 mm und einer Wandstärke von 1 bis 3 mm mitverwendet wird.

2. Verfahren der großtechnischen heterogen katalysierten Gasphasenoxidation von Propan zu Acrolein in einem Oxidationsreaktor, dessen Beschickungsgasgemisch neben Propan und molekularem Sauerstoff als Oxidationsmittel höchstens noch ein inertes Verdünnungsgas umfaßt und dessen Beschickungsgasgemisch zu mehr als 70 Vol.-% aus Propan und zu wenigstens 5 Vol.-% aus molekularem Sauerstoff besteht, **dadurch gekennzeichnet, daß** als Katalysator eine Multimetalloxidmasse der allgemeinen Formel II

$$Mo_{a'} Bi_{b'} X^5_{c'} X^6_{d'} X^7_{e'} O_{f'} \qquad (II),$$

mit

$X^5 =$ V, Nb, Ce, Ta, Fe, Ga und/oder P,
$X^6 =$ Ag, Li, Na, K, Rb, Cs, Tl, Pd, Pt, Au, Cu, Pb und/oder Te,
$X^7 =$ Si, Al, Ti und/oder Zr,
$a' =$ 0,2 bis 2,
$b' =$ 0,3 bis 1,5,
$c' =$ 0 bis 2,
$d' =$ 0 bis 0,5
$e' =$ 0 bis 20 und
$f' =$ eine von Null verschiedene Zahl, die durch die Wertigkeit und Häufigkeit der von Sauerstoff verschiedenen Elemente in II bestimmt wird,

geformt zu einem Vollkatalysatorhohlzylinder mit einem Außendurchmesser und einer Länge von 2 bis 10 mm und einer Wandstärke von 1 bis 3 mm mitverwendet wird.

**3.** Verfahren der großtechnischen heterogen katalysierten Gasphasenoxidation von Propan zu Acrolein in einem Oxidationsreaktor, dessen Beschickungsgasgemisch neben Propan und molekularem Sauerstoff als Oxidationsmittel höchstens noch ein inertes Verdünnungsgas umfaßt und dessen Beschickungsgasgemisch zu mehr als 70 Vol.-% aus Propan und zu wenigstens 5 Vol.-% aus molekularem Sauerstoff besteht, **dadurch gekennzeichnet, daß** als Katalysator eine Multimetalloxidmasse der allgemeinen Formel III

$$X^8_1 P_{a''} X^9_{''b} X^{10}_{c''} O_{d''} \qquad (III),$$

mit

$X^8 =$ V, Nb, Ta, Cr, Mo und/oder W,
$X^9 =$ Sn, Sb, Bi, Te, Fe, Co, Ni, Cu, La und/oder Ce,
$X^{10} =$ Li, Na, K, Rb und/oder Cs,
$a'' =$ 1 bis 10,
$b'' =$ 0 bis 0,5,
$c'' =$ 0 bis 0,5 und
$d'' =$ eine von Null verschiedene Zahl, die durch die Wertigkeit und Häufigkeit der von Sauerstoff verschiedenen Elemente in III bestimmt wird,

geformt zu einem Vollkatalysatorhohlzylinder mit einem Außendurchmesser und einer Länge von 2 bis 10 mm und einer Wandstärke von 1 bis 3 mm mitverwendet wird.

**Revendications**

**1.** Procédé industriel pour l'oxydation en phase gazeuse du propane en acroléine par catalyse hétérogène, où le mélange gazeux d'alimentation contient, outre le propane et de l'oxygène moléculaire en tant qu'agent d'oxydation, au maximum encore un gaz de dilution inerte, et où le mélange gazeux d'alimentation est constitué de plus de 70% en volume de propane et d'au moins 5% en volume d'oxygène moléculaire, **caractérisé en ce que** l'on utilise simultanément comme catalyseur une masse de plusieurs oxydes métalliques de la formule générale I

$$Mo_a Bi_b P_c X^1_d X^2_e X^3_f X^4_g O_h \qquad (I),$$

avec

$X^1 =$ V, Nb, Ta, Cr, W, Ga, Ce et/ou La,
$X^2 =$ Li, Na, K, Rb, Cs, Cu, Ag, Au, Pd et/ou Pt,
$X^3 =$ Sn, Pb, Sb, Bi, Te, Fe, Co et/ou Ni,
$X^4 =$ Si, Al, Ti et/ou Zr,

$$
\begin{aligned}
a &= 0 \text{ à } 2, \\
d &= 0 \text{ à } 2,
\end{aligned}
\quad\left.\right\}\quad \text{avec la condition que la somme de a et d égale au moins 0,20;}
$$

$$
\begin{aligned}
b &= 0 \text{ à } 1{,}5, \\
c &= 0 \text{ à } 10,
\end{aligned}
\quad\left.\right\}\quad \text{avec la condition que la somme de b et c égale au moins 0,1;}
$$

$e =$ 0 à 0,5,
$f =$ 0 à 0,5,
$g =$ 0 à 20 et

h = un nombre différent de zéro, déterminé par la valence et la fréquence des éléments différents de l'oxygène dans I, façonné en un cylindre creux de catalyseur intégral d'un

diamètre extérieur et d'une longueur de 2 à 10 mm et une épaisseur de paroi de 1 à 3 mm.

2. Procédé industriel pour l'oxydation en phase gazeuse du propane en acroléine par catalyse hétérogène, où le mélange gazeux d'alimentation contient, outre le propane et de l'oxygène moléculaire en tant qu'agent oxydant, encore au maximum un gaz de dilution inerte et où le mélange gazeux d'alimentation est constitué à plus de 70% en volume de propane et à au moins 5% en volume d'oxygène moléculaire, **caractérisé en ce que** l'on utilise simultanément comme catalyseur une masse de plusieurs oxydes métalliques de la formule générale II

$$Mo_a, Bi_b, X^5_c, X^6_d, X^7_e, O_f, \tag{II},$$

avec

$X^5 =$ V, Nb, Ce, Ta, Fe, Ga et/ou P,
$X^6 =$ Ag, Li, Na, K, Rb, Cs, Tl, Pd, Pt, Au, Cu, Pb et/ou Te,
$X^7 =$ Si, Al, Ti et/ou Zr,
a' = 0,2 à 2,
b' = 0,3 à 1,5,
c' = 0 à 2,
d' = 0 à 0,5,
e' = 0 à 20 et
f = un nombre différent de zéro, déterminé par la valence et la fréquence des éléments différents de l'oxygène dans II,

façonné en un cylindre creux de catalyseur intégral d'un diamètre extérieur et d'une longueur de 2 à 10 mm et une épaisseur de paroi de 1 à 3 mm.

3. Procédé industriel pour l'oxydation en phase gazeuse du propane en acroléine par catalyse hétérogène, où le mélange gazeux d'alimentation contient, outre le propane et de l'oxygène moléculaire en tant qu'agent oxydant, encore au maximum un gaz de dilution inerte et où le mélange gazeux d'alimentation est constitué à plus de 70% en volume de propane et à au moins 5% en volume d'oxygène moléculaire, **caractérisé en ce que** l'on utilise simultanément comme catalyseur une masse de plusieurs oxydes métalliques de la formule générale III

$$X^8_1 P_{a''} X^9_{b''} X^{10}_{c''} O_{d''} \tag{III},$$

avec

$X^8 =$ V, Nb, Ta, Cr, Mo et/ou W,
$X^9 =$ Sn, Sb, Bi, Te, Fe, Ci, Ni, Cu, La et/ou Ce,
$X^{10} =$ Li, Na, K, Rb et/ou Cs,
a" = 1 à 10,
b" = 0 à 0,5,
c" = 0 à 0,5 et
d" = un nombre différent de zéro, déterminé par la valence et la fréquence des éléments différents de l'oxygène dans III,

façonné en un cylindre creux de catalyseur intégral d'un diamètre extérieur et d'une longueur de 2 à 10 mm et une épaisseur de paroi de 1 à 3 mm.

**Claims**

1. A process for the industrial heterogeneously catalyzed gas-phase oxidation of propane to acrolein in an oxidation reactor which is fed with a gas mixture comprising, apart from propane and molecular oxygen as oxidant, at most one inert diluent gas, and comprising more than 70 % by volume of propane and at least 5 % by volume of molecular oxygen, wherein the catalyst used is a multimetal oxide composition of the general formula I

$$Mo_a Bi_b P_c X^1{}_d X^2{}_e X^3{}_f X^4{}_g O_h \qquad (I),$$

where

$X^1 =$ V, Nb, Ta, Cr, W, Ga, Ce and/or La,
$X^2 =$ Li, Na, K, Rb, Cs, Cu, Ag, Au, Pd and/or Pt,
$X^3 =$ Sn, Pb, Sb, Bi, Te, Fe, Co and/or Ni,
$X^4 =$ Si, Al, Ti and/or Zr,

$$
\begin{aligned}
a &= 0 - 2, \\
d &= 0 - 2,
\end{aligned}
\left.\right\} \text{ with the proviso that the sum of a and d is at least 0.20;}
$$

$$
\begin{aligned}
b &= 0 - 1.5, \\
c &= 0 - 10,
\end{aligned}
\left.\right\} \text{ with the proviso that the sum of b and c is at least 0.1;}
$$

$e =$ 0 - 0.5,
$f =$ 0 - 0.5,
$g =$ 0 - 20 and
$h =$ a number different from zero which is determined by the valence and frequency of the elements different from oxygen in I,

shaped to give an unsupported catalyst in the form of a hollow cylinder having an external diameter and length of from 2 to 10 mm and a wall thickness of from 1 to 3 mm.

2. A process for the industrial heterogeneously catalyzed gas-phase oxidation of propane to acrolein in an oxidation reactor which is fed with a gas mixture comprising, apart from propane and molecular oxygen as oxidant, at most one inert diluent gas, and comprising more than 70 % by volume of propane and at least 5 % by volume of molecular oxygen, wherein the catalyst used is a multimetal oxide composition of the general formula II

$$Mo_a, Bi_b, X^5{}_c, X^6{}_d, X^7{}_e, O_f, \qquad (II),$$

where

$X^5 =$ V, Nb, Ce, Ta, Fe, Ga and/or P,
$X^6 =$ Ag, Li, Na, K, Rb, Cs, Tl, Pd, Pt, Au, Cu, Pb and/or Te,
$X^7 =$ Si, Al, Ti and/or Zr,
$a' =$ 0.2 - 2,
$b' =$ 0.3 - 1.5,
$c' =$ 0 - 2,
$d' =$ 0 - 0.5
$e' =$ 0 - 20 and
$f' =$ a number different from zero which is determined by the valence and frequency of the elements different from oxygen in II,

shaped to give an unsupported catalyst in the form of a hollow cylinder having an external diameter and length of from 2 to 10 mm and a wall thickness of from 1 to 3 mm.

3. A process for the industrial heterogeneously catalyzed gas-phase oxidation of propane to acrolein in an oxidation reactor which is fed with a gas mixture comprising, apart from propane and molecular oxygen as oxidant, at most one inert diluent gas, and comprising more than 70 % by volume of propane and at least 5 % by volume of molecular oxygen, wherein the catalyst used is a multimetal oxide composition of the general formula III

$$X^8_1 P_{a''} X^9_{b''} X^{10}_{c''} O_{d''} \qquad \text{(III)},$$

where

$X^8 =$     V, Nb, Ta, Cr, Mo and/or W,
$X^9 =$     Sn, Sb, Bi, Te, Fe, Co, Ni, Cu, La and/or Ce,
$X^{10} =$    Li, Na, K, Rb and/or Cs,
$a'' =$      1 - 10,
$b'' =$      0 - 0.5,
$c'' =$      0 - 0.5 and
$d'' =$      a number different from zero which is determined by the valence and frequency of the elements different from oxygen in III,

shaped to give an unsupported catalyst in the form of a hollow cylinder having an external diameter and length of from 2 to 10 mm and a wall thickness of from 1 to 3 mm.